Europäisches Patentamt

European Patent Office

Office européen des brevets

(11) Publication number: **0 483 500 A1**

# EUROPEAN PATENT APPLICATION

(21) Application number: **91115735.2**

(22) Date of filing: **17.09.91**

(51) Int. Cl.5: **A61K 7/16**

(30) Priority: **31.10.90 US 606371**

(43) Date of publication of application:
**06.05.92 Bulletin 92/19**

(84) Designated Contracting States:
**AT BE CH DE DK ES FR GB IT LI LU NL SE**

(71) Applicant: **Colgate-Palmolive Company**
**300 Park Avenue**
**New York, N.Y. 10022-7499(US)**

(72) Inventor: **Thomas, Kathleen P.**
**11 Tall Oaks Road**
**Somerset, N.J.(US)**

(74) Representative: **UEXKÜLL & STOLBERG**
**Patentanwälte**
**Beselerstrasse 4**
**W-2000 Hamburg 52(DE)**

(54) **Odor absorbing articles.**

(57) An article for adsorbing mouth odors includes a packet containing an activated charcoal, zeolite, silica or alumina for placing in the mouth. The packet is formed from a porous substantially insoluble material. The packet is placed in the mouth for a period of time to adsorb mouth odors and then discarded.

## FIG. I

## Field of the Invention

The present invention is directed to an article adapted for placing in the mouth to remove mouth odors. More particularly the invention relates to a disposable packet containing a composition for absorbing odors and for releasing a flavor or treating composition to the mouth.

## Background of the Invention

Compositions are well know for masking mouth odors. These compositions may be in the form of liquids, aerosol sprays or solids, such as mouth washes, breath sprays, chewing gums, tablets and the like. The previous efforts for the control of mouth and breath odors are generally related to providing the sustained release of a medicament or odor masking composition.

The previous compositions and products have not been effective in providing sustained release of medicaments or compositions that are suitable for masking mouth odors. Most liquid sprays and mouth washes provide only a short period of time in which the medicament or breath freshener remains active the mouth. Liquid sprays and mouth washes generally dissipate and wash away in a relatively short period of time. Tablets and chewing gums also do not provide prolonged delivery of the odor-masking agent or medicament. Tablets typically dissolve quickly in the mouth, and chewing gums quickly release the active component in only a few minutes.

One example of the prior devices to mask mouth odors is disclosed in U.S. Patent No. 3,911,099. This device provides a medicament in liquid or solid form in a sustained release composite. The article is intended to be fixed to the oral mucosa in the mouth by an adhesive to provide a sustained release of an odor masking component. The article is in the form of a tablet or composite of microcapsules which is adhesively secured to the gum or cheek. When the article has completely dissolved, the adhesive portion of the article can be removed. This article has the disadvantage of providing only the release of odor masking substances, and does not remove the odor-producing components from the oral cavity.

Another example of the prior devices is U.S. Patent No. 307,537 disclosing a porous linen bag containing capsicum or other medicaments. The bag is placed in the mouth to externally treat inflamed gums. The bag is provided with an impervious side to prevent irritation of the inside of the cheek and a porous side for contacting the gum surface.

U.S. Patent No. 1,741,589 relates to a chewing gum containing charcoal. The charcoal is slowly released from the gum during chewing and swallowed to aid in digestion. The charcoal is released in a manner to prevent adhering of the charcoal to the teeth to avoid the unpleasant appearance of charcoal in the mouth.

U.S. Patent No. 2,690,415 relates to an odor absorbent body for use as a bandage, surgical dressing, blanket and the like. The material is formed from an open mesh of a woven fabric containing discrete portions of an adhesive. A layer of granular odor absorbent material is then applied to the fabric to provide an odor-adsorbing coating.

The above-noted articles and compositions do not provide for the adequate removal of odors from the mouth, but instead attempt to mask the odors by releasing odor-masking agents. The release of odor-masking components and flavors may mask mouth odors for a short period of time, but do not remove the odor-causing substances such that the odors are again apparent within a short time. There is still a need for an article which is able to remove the odors and odor-causing substances from the mouth.

## Summary of the Invention

The present invention is directed to a porous article which is to be placed in the mouth for a period of time. The article is in the form of a disposable packet or pouch produced from an insoluble porous material. The pouch contains a therapeutic amount of a solid adsorbent material to adsorb odor-producing substances in the mouth. During use, the pouch is placed in the mouth for an effective period of time and then discarded. The pouch further contains flavorants, breath freshening agents, dentifrices, such as antibacterial agents, antifungal agents, fluoride compounds and/or teeth whitening agents.

The packet is formed from a porous material which is insoluble or substantially insoluble in saliva. The material may be a woven or non-woven fabric of fibers of cellulose, cotton, rayon, polyester, nylon or other suitable material. The material may further be paper or paper-like materials. Alternatively, the packet material may be a polymeric film having perforations therein. The pouch is preferably soft and flexible to readily conform to the contour of the mouth, gums or cheek. The packet may alternatively be semi-rigid and contoured to conform to the mouth surfaces. In embodiments of the invention, the packet may have one or more portions covered or coated with a saliva-impervious material to control the rate of release or adsorption of materials. The porous packet may further be coated with a soluble film-forming material to substantially encapsulate the odor-adsorbing compound. The soluble coating is dissolved when the packet is placed in the mouth to expose the porous packet surface.

The odor adsorbent is preferably an insoluble material, for example, an activated charcoal, zeolite, alumina, silica or organomodified silica capable of adsorbing the odor producing substances in the mouth. The contents of the pouch which are to be released from the pouch, such as flavors or medicaments, may be encapsulated or coated with materials to impart sustained release qualities to components. The packet is placed in the mouth to adsorb the odor-causing substances and release the flavor and medicaments. After an effective period of time, the packet is discarded along with the odor-causing substances.

## BRIEF DESCRIPTION OF THE DRAWINGS

The invention is illustrated in the drawings of which the following is a brief description.

Figure 1 is a plan view of the packet in accordance with the preferred embodiment of the invention.

Figure 2 is a plan view of an alternative embodiment illustrating a coating material.

Figure 3 is a plan view of a further embodiment of the invention having a water soluble coating.

Figure 4 is a plan view further embodiment showing a compartmentalized packet.

## DETAILED DESCRIPTION OF THE INVENTION

The disadvantages of the previous sustained flavor release compounds are obviated by the present invention while providing an effective means for eliminating breath and mouth odors. The invention is directed to a disposable pouch or packet having a saliva permeable outer surface. The packet is sealed around all edges and contains a substance for adsorbing odor producing substances from the mouth. The packet may further contain an optional flavorant, dentifrice, or medicament which is released from the packet when placed in the mouth.

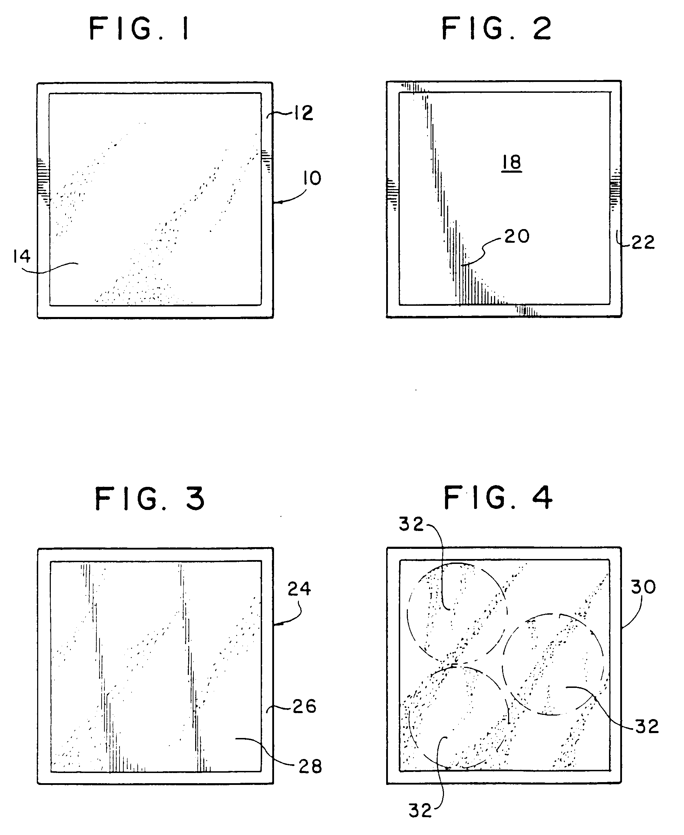

The article of the invention is in the shape of a packet or pouch member 10 shown in Figure 1. The packet preferably has the shape of a square although the pouch may be rectangular, circular or any other shape depending on the intended use and placement of the device in the mouth. The pouch is preferably hollow and of a size to contain a therapeutic amount of an odor adsorbing composition. The pouch is sealed around the periphery by an adhesive or by heat sealing the edges 12 to enclose the contents. The packet is generally formed from two pieces of the porous material and sealed about its edges in the form of an envelope. Alternatively, the packet may be a single piece of material folded in half and sealed around the edges. The packet may further be formed from a tubular shaped material which is sealed at the two open ends of the tube.

The material from which the packet is made is a porous material, such as a woven or non-woven fabric, paper or paper-like material 14. In the preferred embodiment of the invention, the porous material is an inert, tasteless and odorless material such as cotton, cellulose, rayon, jute, polyester, nylon, polyamides and the like. The pore size of the material is generally sufficiently small to retain the contents of the pouch and to allow the flow of saliva into and out of the packet. The packet of material is preferably soft and flexible to allow the packet to conform to the shape of the gum or other portion of the mouth where the pouch is placed, as discussed hereinafter in greater detail.

In further embodiments of the invention the packet may be formed from a non porous film 18 as shown in Figure 2. The packet may be formed from a polymeric film and be provided with perforations 20 over the entire surface area or over selected portions. The packet is formed by heat sealing the peripheral edges 22. The packet may further be produced from laminated materials to inhibit interaction of the contents with the packet material.

The packet contains a therapeutic amount of an insoluble odor adsorbing substance such as, for example, activated charcoal, zeolites, alumina, silica gel, silica and organomodified silica. The specific odor adsorbing component is not critical, provided the component is non-toxic to humans or other animals and does not produce an unpleasant taste in the mouth. The contents of the packet preferably further include a flavorant, sweetener, breath freshener, dentifrice and/or medicinal compound. The dentifrice may include, for example, a fluoride-containing compound, teeth whitener, antiseptic, antifungal or antibacterial agent. Suitable antiseptic components may be, for example, benzothonium chloride, acetyl trimethyl ammonium bromide and sanguinaria. The medicinal compound can also be an antibiotic such as tetracycline, cetyl pyridinium chloride, benzylthonium chloride, chlorhexidine and hexachlorophene. The breath fresheners and flavors may be natural flavors, herbs and spices such as wintergreen, eucalyptus, clove, cinnamon, spearmint, peppermint or oleoresins and extracts of the flavor. Alternatively, the flavor may be a natural or an artificial flavor or sweetener such as aspartame, saccharin, cyclamates and the like. In the preferred embodiment of the invention, the flavorant, medicament or dentifrice is mixed uniformly with the odor adsorbing component. All or a portion of the flavor or other component may be coated on the surface of the packet to provide an immediate release of the flavor component when the device is placed in the mouth.

The flavor, dentifrice or medicament can be mixed with the odor adsorbing component in the substantially pure form. Alternatively, the additive may be encapsulated in microcapsules or in another substrate to provide a sustained release of the component from the packet.

In a further embodiment the outer surface of the packet may further be coated with a portion of the odor adsorbent component by the use of a suitable adhesive. The outer surface of the pouch is preferably porous to saliva to allow the ready transfer of saliva through the wall of the bag. A coating of a suitable moisture-proofing material may be applied to a portion of the packet to reduce the surface area of the porous portion, thereby controlling the overall porosity of the pouch and the rate of odor adsorption and release of the flavor, dentifrice or medicament from the pouch. In a further embodiment, one face of the pouch may be made of a moisture impervious material and the other face made from a porous material to provide controlled adsorption of odor-causing substances and release of the other components.

In a further embodiment shown in Figure 3, the packet 24 is formed from two pieces of porous fabric sealed around its edges 26 to form an envelope and filled with an odor-adsorbing compound. All or a portion of the packet is coated with a water soluble film-forming component 28. The entire packet may be coated with the film-forming component to substantially fill the interstices of the fabric and to substantially encapsulate the contents of the packet. The film-forming component provides a moisture and oxygen impervious barrier to the contents of the packet during storage to extend the shelf life until the packet is placed in the mouth. When the packet is placed in the mouth, the soluble film-forming coating will dissolve to allow the saliva to transfer into and out of the packet and to allow the flavor, dentifrice or medicament to diffuse from the packet. The rate at which the coating dissolves to expose the contents of the packet may be controlled by the coat weight of the coating and the type of film-forming component.

The coating of the film-forming component may be continuous and uniformly applied to the surface of the packet. Alternatively, the coating may be discontinuous and cover only selected portions of the packet. The coating may further be nonuniform such that portions may dissolve faster to expose selected portions of the contents of the packets to the saliva for release of components or adsorption of odor-causing substances in the saliva.

The film-forming material may be a material that is soluble in saliva and is substantially tasteless and odorless. The film-forming material may include, for example, water soluble gums, such as acacia gum, xanthan gum or gum tragacanth, ethylcellulose, carboxymethyl cellulose, cellulose acetate phthalate, hydroxypropyl cellulose, hydroxyethyl cellulose, shellac, polyvinylalcohol, polyvinylacetate or combinations thereof. The coating of the film-forming material may contain a flavor, dentifrice, medicament or an odor-absorbing material dispersed therein.

In the preferred embodiment, the pouch is a size suitable to fit comfortably in the mouth, such as under the tongue or in the cheek. Typically, the packet is about one half inch square to about one inch by one inch. The packet may be a square, rectangular, circular or tubular shape. The packet is preferably flexible and deformable to conform to the contour of the mouth to fit comfortably without interfering with speech. In an alternative embodiment, the pouch is semi-rigid and preformed to fit the curvature of the mouth. The pouch can be placed in the mouth after eating, smoking or drinking for a period of time to adsorb the odor producing components from the saliva. After an effective treatment time, the pouch and the odor adsorbing compound is removed from the mouth and discarded, thus removing the odor-causing substances from the mouth.

In alternative embodiments, the odor adsorbing article is a solid bundle or mass of fibers of cellulose or polymeric fibers having the odor adsorbent and a flavorant, dentifrice or medicament intermixed with the fibrous mass. The odor-absorbing component is preferably intermixed with the fibers and shaped to form a bundle and to inhibit loss of the odor absorbing material from the bundle. In this manner the odor-adsorbing component and the adsorbed odor-causing substances are removed from the mouth and discarded.

In a further embodiment illustrated in Figure 4, the packet 30 may be compartmentalized to form two or more porous compartments such that the odor-adsorbing component is separate from the dentifrice, flavorant and medicament. Separating the components may inhibit interaction of the components and loss of efficacy prior to placing the packet in the mouth. The porosity of the packet may be adjusted as desired to selectively control the rate of flow of saliva through the wall of the packet into each of the compartments, thereby controlling the rate of odor adsorption and release of components. For example, the rate of porosity of a first compartment containing a flavorant may be greater than other compartments containing the odor absorbing component. The porosity of the compartments may be controlled by selecting the type of fabric material, pore size, or by applying the coating of the film-forming compartment at differing coat weights to the different compartments.

The packet may be formed having distinct compartments. Alternatively, the packet 30 may include a plurality of separate and distinct smaller packets 32 of each desired component which are contained in a second larger packet as shown in Figure 4. Each smaller packet may have individually selected porosity and rate of transfer of saliva to control and adjust the adsorption of odor causing substances and release of dentifrice, medicament and/or flavorant. Separating the components into different packets or compartments allows the use of components which are not compatible and which can not usually be incorporated in the same container

The above description is intended to disclose the preferred embodiments of the invention. It will be readily understood by those skilled in the art that numerous modification and embodiments can be practiced by one skilled in the art without departing from the spirit and scope of the invention.

**Claims**

1. An odor-adsorbent article for placing in the mouth for adsorbing mouth odors comprising a porous, substantially insoluble flexible packet containing at least one substantially insoluble odor-adsorbing agent, wherein said odor-absorbing agent is capable of adsorbing odor causing substances from saliva.

2. The article of claim 1 wherein said odor-adsorbing agent is selected from the group consisting of activated charcoal, zeolites, silica, alumina and mixtures thereof.

3. The article of claim 1 wherein said packet is formed from woven or non-woven fabric selected from the group consisting of cotton, cellulose, polyester, rayon, nylon, polyamides and mixtures thereof.

4. The article of claim 1 wherein the packet is formed from a perforated insoluble film.

5. The article of claim 1 wherein the packet further contains a component selected from the group consisting of flavorants, dentifrices, medicaments and mixtures thereof.

6. The article of claim 1 wherein at least a portion of the packet is substantially nonporous to saliva.

7. The article of claim 1 wherein the packet has a first porous side and a second nonporous side.

8. The article of claim 1 wherein said packet includes an outer coating of a flavorant.

9. The article of claim 1 wherein said odor-adsorbing agent is contained within a first porous packet and a second component is contained in a second porous packet, and wherein said first and second packets are contained in a third porous packet.

10. An odor-adsorbing article for placing in the mouth of an animal to adsorb mouth odors and odor-producing substances from the mouth comprising a porous, substantially insoluble packet containing at least one substantially insoluble odor-adsorbing agent, wherein said packet is porous to allow the transfer of saliva, and wherein at least a portion of the surface of said packet is provided with an outer coating of a water soluble film-forming component to substantially inhibit the flow of saliva through said packet to said odor-adsorbing agent, said film-forming component being sufficiently soluble in saliva to enable transfer of saliva through said packet when said film-forming component has dissolved.

11. The article of claim 10 wherein said odor-adsorbing agent is selected from the group consisting of activated charcoal, zeolites, silica, alumina and mixtures thereof.

12. The article of claim 10 wherein said packet is formed from porous woven or non-woven fabric, wherein interstices of said fabric are filled with said soluble film-forming component to substantially encapsulate said odor-adsorbing agent.

13. The article of claim 10 wherein said outer coating of said film-forming component is substantially continuous thereby substantially encapsulating said odor-adsorbing agent.

14. The article of claim 10 wherein said outer coating of said film-forming component is discontinuous.

**15.** The article of claim 10 wherein said outer coating includes a component selected from the group consisting of odor adsorbing agents, flavors, dentifrices, medicaments and mixtures thereof.

**16.** The article of claim 10 wherein the content of said packet further contains at least one component selected from the group consisting of flavorants, dentifrices and medicaments.

**17.** The article of claim 10 wherein the outer coating of said film-forming component is non-uniform to sectively expose areas of the surface of the packet at different rates as the coating is dissolved whereby the odor-adsorbing rate is sustained.

**18.** The article of claim 10 wherein said packet is compartmentalized and wherein said odor-absorbing agent is in a first compartment and at least one component selected from the group consisting of flavors, dentifrices and medicament is in a second compartment of said packet.

**19.** The article of claim 18 wherein said first and second compartments comprise first and second porous packets contained within a third porous packet.

**20.** The article of claim 10 wherein said outer coating of a water soluble film-forming component is selected from the group consisting of acacia gum, xanthan gum, gum tragacanth, ethylcellulose, carboxymethyl cellulose, cellulose acetate phthalate, hydroxypropyl cellulose, hydroxyethyl cellulose, shellac, polyvinylalcohol, polyvinylacetate and mixtures thereof.

**21.** A disposable odor-adsorbing article for placing in the mouth of an animal to adsorb mouth odors and odor-producing substances comprising a first porous, substantially insoluble packet containing an insoluble odor-adsorbing agent, and a second porous insoluble packet containing a component selected from the group consisting of dentifrices, flavors, medicaments and mixtures thereof, wherein the first and second packets have a porosity to provide sustained release of the contents of the packets, and wherein said first and second packets are contained within a third porous packet.

**22.** The article of claim 21 wherein said first and second packets include an outer coating of a water soluble film-forming component selected from the group consisting of acacia gum, xanthan gum, gum tragacanth, ethylcellulose, carboxymethyl cellulose, cellulose acetate phthalate, hydroxypropyl cellulose, hydroxyethyl cellulose, shellac, polyvinylalcohol, polyvinylacetate and mixtures thereof.

# FIG. 1

# FIG. 2

# FIG. 3

# FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | WPIL, FILE SUPPLIER, AN=90-189686, Derwent Publications Ltd, London, GB; & JP-A-02 124 812 (KOKUSAI CHITEKI SHO) * Abstract * | 1-22 | A 61 K   7/16 |
| A | WO-A-9 006 745  (COPELAN et al.) * Claim 1 * | 1-22 | |
| A | US-A-4 900 552  (SANVORDEKER et al.) * Whole document * | 1-22 | |
| A | EP-A-0 392 528  (KIMBERLEY-CLARK CORP.) * Whole document * | 1-22 | |
| A | EP-A-0 158 092  (HENKEL KgaA) * Page 8, line 29 - page 9, line 19; claims * | 1,3,5 | |
| D,A | US-A-2 690 415  (F.A. SHULER) * Whole document * | 1,3 | |

TECHNICAL FIELDS SEARCHED (Int. Cl.5)

A 61 K
A 61 L

The present search report has been drawn up for all claims

| Place of search | Date of completion of the search | Examiner |
|---|---|---|
| THE HAGUE | 07-11-1991 | COUCKUYT P.J.R. |